## Europäisches Patentamt
## European Patent Office
## Office européen des brevets

(19)

(11) **EP 0 795 127 B1**

(12) **EUROPÄISCHE PATENTSCHRIFT**

(45) Veröffentlichungstag und Bekanntmachung des Hinweises auf die Patenterteilung:
**06.10.1999 Patentblatt 1999/40**

(21) Anmeldenummer: **95941040.8**

(22) Anmeldetag: **28.11.1995**

(51) Int Cl.[6]: **G01N 27/417**, G01N 27/407

(86) Internationale Anmeldenummer:
**PCT/EP95/04687**

(87) Internationale Veröffentlichungsnummer:
**WO 96/17242 (06.06.1996 Gazette 1996/26)**

(54) **VORRICHTUNG UND VERFAHREN ZUR BESTIMMUNG VON GASFÖRMIGEN BESTANDTEILEN IN GASGEMISCHEN**

DEVICE AND PROCESS FOR DETERMINING GASEOUS COMPONENTS OF GAS MIXTURES

DISPOSITIF ET PROCEDE DE DETERMINATION DE CONSTITUANTS GAZEUX DANS DES MELANGES GAZEUX

(84) Benannte Vertragsstaaten:
**DE ES FR GB IT SE**

(30) Priorität: **28.11.1994 DE 4442272**

(43) Veröffentlichungstag der Anmeldung:
**17.09.1997 Patentblatt 1997/38**

(73) Patentinhaber: **Heraeus Electro-Nite International N.V.**
**3530 Houthalen (BE)**

(72) Erfinder:
• **SCHÖNAUER, Ulrich**
**D-76131 Karlsruhe (DE)**
• **GÖPEL, Wolfgang**
**D-72076 Tübingen (DE)**
• **REINHARDT, Götz**
**D-72074 Tübingen (DE)**

• **SOMOV, Serguie**
**Ekaterinenburg, 620151 (RU)**
• **GUTH, Ulrich**
**D-17491 Greifswald (DE)**
• **BROSDA, Susanne**
**D-17493 Greifswald (DE)**

(74) Vertreter: **Kühn, Hans-Christian et al**
**Heraeus Holding GmbH,**
**Stabsstelle Schutzrechte,**
**Heraeusstrasse 12-14**
**63450 Hanau (DE)**

(56) Entgegenhaltungen:
**EP-A- 0 517 366          EP-A- 0 533 991**
**EP-A- 0 678 740          WO-A-92/14143**
**DE-A- 3 742 014          GB-A- 2 288 873**
**US-A- 4 285 790          US-A- 5 049 254**
**US-A- 5 397 442**

**Beschreibung**

Die vorliegende Erfindung betrifft eine Vorrichtung zur Bestimmung der Konzentrationen von gasförmigen Bestandteilen in Gasgemischen einschließlich Sauerstoff sowie ein Verfahren zur Bestimmung der Konzentrationen von zumindest einem der gasförmigen Bestandteile in solchen Gasgemischen.

Aus US-A-5.049.254 ist eine Vorrichtung zur Bestimmung der Konzentration von Kohlendioxid und Wasser durch Zersetzung bekannt. Kohlendioxid und Wasser werden gleichzeitig zersetzt. Eine selektive Zersetzung der Gaskomponenten ist mit dieser Anordnung nicht möglich. DE 37 42 014 A1 offenbart einen Gassensor zur gleichzeitigen Bestimmung mehrerer Gaskomponenten. Eine ähnliche Anordnung ist aus US-A-4.285.790 bekannt. Das zu messende Gas erreicht durch einen zentralen Zugang zum Messraum die Elektroden gleichzeitig, so daß Quereinflüsse nicht vermieden werden können. Aus WO-A-96/14575 ist ein Gassensor bekannt zur Messung von Stickoxiden und Sauerstoff. Dabei ist die Sauerstoffelektrode mit einer für Stickoxide undurchlässigen Beschichtung versehen, um lediglich den Sauerstoffanteil des Gasgemisches abzupumpen und an der zweiten Elektrode sauerstoffbefreites Gas zur Verfügung zu stellen.

Es ist eine solche, Multisensorsystem genannte Vorrichtung an sich bekannt (WO 92/14143). Hierbei sind mehrere potentiometrische Sensoren auf einem gemeinsamen Träger, der auch zugleich der sauerstoffionenleitende Festelektrolyt sein kann, angebracht. Mit diesen verschiedenen Sensoren können gleichzeitig die verschiedenen gasförmigen Bestandteile des Gasgemisches gemessen werden.

Aufgrund der nicht idealen Selektivität der Elektroden der Sensoren und von Interdiffusionseffekten kann es zu Quereinflüssen zwischen den Elektroden verschiedener Sensoren kommen. Insbesondere wird mit zunehmendem Anteil von Sauerstoff das Meßergebnis anderer gasförmiger Bestandteile des Gasgemisches verfälscht und führt, was nicht erwünscht ist, zu ungenauen Meßergebnissen.

Der Erfindung liegt deshalb die Aufgabe zugrunde, eine Vorrichtung sowie ein Verfahren zur Bestimmung von gasförmigen Bestandteilen so weiter auszugestalten, daß die verschiedenen gasförmigen Bestandteile des Gasgemisches gleichzeitig genauer gemessen werden können.

Diese Aufgabe wird gemäß den Merkmalen des Vorrichtungssowie Verfahrenshauptanspruchs gelöst.

Bei konstanten Umgebungsbedingungen wird durch die Diffusionsbarriere ein konstanter Volumenstrom des zu messenden Gasgemisches in den Diffusionskanal eingestellt. Das in Längenrichtung des Diffusionskanals erste Elektrodenpaar ist, da es amperometrisch geschaltet ist, als sogenannte Sauerstoffpumpe ausgebildet, bei der der in den Diffusionskanal gelangende molekulare Sauerstoff an der inneren Elektrode in Sauerstoffionen dissoziiert wird, welche letztere dann aufgrund des am Elektrodenpaar angelegten Potentials in dem sauerstoffionenleitenden Festelektrolyt aus dem Diffusionskanal heraustransportiert werden. Hierbei stellt sich ein elektrischer Grenzstrom ein, der unabhängig von der an die beiden Elektroden angelegten elektrischen Spannung ist und nur vom Sauerstoffpartialdruck des zu messenden Gasgemisches abhängig ist. Letzterer kann durch Messung des elektrischen Grenzstroms bestimmt werden. Hinter dem ersten Elektrodenpaar liegt somit das Gasgemisch mit einer definierten Konzentration von Sauerstoff oder ohne denselben vor. Die in Längenrichtung sich anschließenden weiteren Elektrodenpaare können nunmehr amperometrisch oder potentiometrisch geschaltet sein. An ihnen können die betreffenden gasförmigen Bestandteile des Gasgemisches nunmehr ohne Einfluß von Sauerstoff genauer und mit besserer Signalstärke gemessen werden. Es kann hierbei auch eines der weiteren Elektrodenpaare dazu verwendet werden, Sauerstoffionen an die innere dem zu messenden Gasgemisch zugewandte Elektrode zu transportieren und dort eine Oxidation, z.B. von Stickstoff- oder Kohlenmonoxid zu Stickstoff- bzw. Kohlendioxid durchzuführen, wobei diese gasförmigen Bestandteile an einem der weiteren potentiometrischen oder amperometrischen Sensoren gemessen werden können. Dabei können, wenn Sauerstoff gänzlich aus dem Gasgemisch entfernt worden ist an Sensoren, die gegenüber Sauerstoff querempfindlich sind, Störungen durch den Sauerstoffgehalt nicht auftreten. Querempfindlichkeiten weiterer Sensoren gegenüber einzelnen gasförmigen Bestandteilen, die eine Messung ungünstig beeinflussen würden, lassen sich aber bei geeigneter Auswahl der Sensormaterialien oder des Sensoraufbaus auch durch andere Effekte ausschalten, beispielsweise durch Hydrations-, Polarisations-, Belegungseffekte oder dgl.. Die weiter oben diskutierten amperometrisch geschaltete Gassensoren sind hierbei an sich bekannt (DE-38 11 713 A1; DE-41 07 217 C2).

Zweckmäßige Ausgestaltungen und Weiterbildungen der Erfindung sind in den Unteransprüchen gekennzeichnet. Erläuternd sei auf folgendes hingewiesen: Festelektrolytsensoren beruhen generell auf der direkten Umwandlung chemischer freier Reaktionsenthalpie in elektrische Energie, die beispielsweise bei den potentiometrischen Sensoren zu einer sich ergebenden Potentialdifferenz führt, oder auf einer durch Anwendung elektrischer Energie erzwungenen chemischen Reaktion, wie sie beispielsweise bei amperometrischen oder polarographischen Sensoren eingesetzt wird. Dabei können bei allen diesen Sensortypen entweder Reaktionen an der Elektrode zwischen dem Gas und den im Elektrolyten wandernden oder unbeweglichen Ionen mit einer komplexen, über Zwischenphasen modifizierten Reaktion zugrundeliegen.

Erfindungsgemäß können als weitere sowohl potentiometrische als auch amperometrische Sensoren verwendet werden. Bei den potentiometrischen Sensoren wird das Potential einer dem zu messenden Gasgemisch ausgesetzten Elektrode gegenüber dem Potential einer Referenzelektrode stromlos gemessen. Bei den klassischen potentiometrischen Sensoren steht dabei das Gas mit dem Ionen des Festelektrolyten oder mit der Zusammensetzung des Festelektrolyten im Gleichgewicht. Die Größe der Potentialdifferenz ist eine eindeutige Funktion der Konzentration des betreffenden Gases. Eine Kalibrierung des Sensors ist in der Regel überflüssig, weil sich im Idealfall ein elektrochemisches Gleichgewicht einstellt.

Diese klassischen Sensoren zeigen jedoch in fließenden Gassystemen, die nicht im thermodynamischen Gleichgewicht sind, Querempfindlichkeiten zu anderen Gasen, was zu Potentialverschiebungen führt. Ursache hierfür sind katalytische Effekte der Elektroden an der Phasengrenze zwischen der Elektrode und dem Elektrolyten, wo neben den Elektrodenreaktionen zusätzliche elektrochemische Reaktionen stattfinden können.

Bei potentiometrischen Sensoren mit Festkörperionenleitern sind verschiedene Typen in Gebrauch. Für die Bestimmung von $CO_2$ hat sich ein Festionenleiter aus $Na_2CO_3$-$BaCO_3$, NASICON/$Na_2CO_3$ oder Na-β-Alumina/$Na_2CO_3$ als geeignet erwiesen. Für die Bestimmung von Sauerstoff wird im allgemeinen mit Yttrium oder Calcium stabilisiertes Zirkondioxid verwendet. Für die Bestimmung von $NO_2$ wird ein Festionenleiter aus Na-β-β"-Alumina/$NaNO_3$ oder $Ba(NO_3)_2$ oder NASICON/$NaNO_2$ verwendet. Der letztere Festionenleiter ist auch zur Bestimmung von NO verwendbar. Zur Bestimmung von Fluor wird z.B. $LaF_3$, zur Bestimmung von $Cl_2$, z.B. ein Festionenleiter aus AgCl und zur Bestimmung von $SO_x$ z.B. ein Festionenleiter aus $K_2SO_4$, NASICON/$Na_2SO_4$ oder $Ag_2SO_4$-$Li_2SO_4$ verwendet.

Bei amperometrischen Sensoren werden über eine angelegte elektrische Spannung Ionen durch den Festelektrolyten gepumpt. Der Antransport wird über an sich bekannte Diffusionsbarrieren, z.B. in Form von porösen Schichten oder Kapillaren gehemmt, wodurch ein elektrischer Grenzstrom auftritt, der proportional zur Konzentration des betreffenden Gases ist und nicht mehr von der Erhöhung der elektrischen Spannung abhängt. Amperometrische Sauerstoffsensoren sind vor allen Dingen zur Messung des Sauerstoffpartialdrucks im Abgas von Magermotoren, die mit einem geringen Sauerstoffüberschuß arbeiten, verwendet worden, da der gemessene Strom linear vom Sauerstoffpartialdruck abhängt und damit geringe Änderungen gut nachweisbar sind. Die logarithmische Charakteristik der potentiometrischen Sensoren ist für diese Anwendung wenig geeignet.

Bei höheren Reduktionspotentialen können neben Sauerstoff auch andere sauerstoffhaltige Komponenten, wie $CO_2$ oder $H_2O$, reduziert werden. Damit werden auch diese Gase einer amperometrischen Messung zugänglich.

Als Basismaterial für den eingesetzten Festelektrolyten soll ein sauerstoffionenleitender Festelektrolyt eingesetzt werden. Hierzu hat sich mit Yttrium stabilisiertes Zirkondioxid als besonders geeignet erwiesen. Dabei soll der Festelektrolyt an einen Diffusionskanal angrenzen, durch den das Gas mit den zu bestimmenden Einzelkomponenten hindurchdiffundiert. Die einzelnen Gaskomponenten werden bei einer Ausführungsform der Erfindung sequentiell an in Längenrichtung des Diffusionskanals aufeinanderfolgenden Elektroden quantitativ umgesetzt. Nach dem Grundprinzip der Amperometrie ist dabei der fließende Strom durch jede Elektrode im wesentlichen proportional zur Konzentration der jeweiligen Komponente in der Gasphase. Es kann aber wegen der nicht idealen Selektivität der Elektroden und Interdiffusionseffekten zu Quereinflüssen zwischen den Elektroden kommen. Ein besonderer Vorteil der Anordnung liegt in der Veränderung der Gasphasenzusammensetzung im Verlauf der Diffusion des Gases von Elektrode zu Elektrode. Dadurch wird der Einfluß der wesentlichen Störgase unterdrückt. Die einzelnen Gase lassen sich über folgende Elektrodenreaktionen nachweisen:

Sauerstoff:

$$O_2 + 4e^- (El) \rightarrow 2O^{2-} (ZrO_2) \qquad (1)$$

Stickoxide($NO_x$):

1. Oxidation von NO zu $NO_2$:

$$NO + O^{2-}(ZrO_2) \rightarrow NO_2 + 2e^-(El) \qquad 2)$$

2. Reduktion von $NO_2$ zu NO:

$$NO_2 + 2e^-(El) \rightarrow NO + O^{2-}(ZrO_2) \qquad (3)$$

3. Reduktion von NO:

$$NO + 2e^-(El) \rightarrow 1/2N_2 + O^{2-}(ZrO_2) \qquad (4)$$

$CO_x$:

1. Oxidation von CO zu $CO_2$:

$$CO + O^{2-}(ZrO_2) \rightarrow CO_2 + 2e^-(El) \qquad (5)$$

2. Reduktion von $CO_2$ zu CO:

$$CO_2 + 2e^-(El) \rightarrow CO + O^{2-}(ZrO_2) \qquad (6)$$

$SO_x$:

1. Oxidation von $SO_2$:

$$SO_2 + O^{2-}(ZrO_2) \rightarrow SO_3 + 2e^-(El) \qquad (7)$$

2. Reduktion von $SO_3^-$:

$$SO_3^- + e^-(El) \rightarrow SO_2 + O^{2-}(ZrO_2) \qquad (8)$$

3. Reduktion von $SO_2$:

$$SO_2 + 4e^-(El) \rightarrow 1/2 S_2 + 2O^{2-}ZrO_2 \qquad (9)$$

$C_yH_x$:

$$C_yH_x + (x/2 + 2y)O^{2-}(ZrO_2) \rightarrow$$
$$\rightarrow yCO_2 + x/2*H_2O + (4y + x)e^-(El) \qquad (10)$$

In der Vorrichtung wird zunächst in einem ersten Schritt in einem sauerstoffreichen Abgas, z.B. Diesel- oder Gasbrennerabgas, mit einer ersten Elektrode der Sauerstoff gemäß der oben angegebenen Formel (1) für die Reduktion von Sauerstoff entfernt. Danach können die übrigen Komponenten des Abgases, beispielsweise $NO_x$ oder $SO_x$, an den nachfolgenden Elektroden bestimmt werden.

Die Elektroden, insbesondere die Elektroden der ersten Meßanordnung, sollen eine gute Sauerstoffaustauschkinetik aufweisen, damit beispielsweise eine vollständige Entfernung des Sauerstoffs aus dem Gasgemisch erreicht werden kann. Die Arbeitsbedingungen werden zweckmäßigerweise allerdings hinsichtlich Potential und Temperatur so gehalten, daß eine weitere Reduktion des NO und $SO_2$ vermieden wird. Die verwendete Spannung an der aktiven Elektrode soll dabei aber -400 mV, bezogen auf 1 bar Sauerstoffpartialdruck nicht überschreiten. Geeignet dafür sind Platin oder geeignete Festelektrolyt-Platin-Mischungen oder gemischt leitende Oxidelektroden, wie z.B. $La_{(1-x)}Sr_xCrO_3$-Elektroden, die eine hohe Aktivität für den Sauerstoffaustausch aufweisen.

Bei ausreichender Elektrodenoberfläche der ersten Elektrode und geeigneter Zellanordnung erhält die zweite Elektrode der zweiten Meßanordnung ein sauerstoff-freies Restgas. An dieser Elektrode kann dann beispielsweise die elektrokatalytische Zersetzung des NO stattfinden nach

$$NO + 2e^-(El) = 1/2\, N_2(Gas) + O^{2-}(ZrO_2) \qquad (11)$$

Die Reduktion des NO muß in einem mittleren kathodischen Potentialdifferenzbereich von ca. -400 mV bis -700 mV stattfinden, um eine Reduktion weiterer Komponenten, wie $CO_2$, $SO_2$ und $H_2O$ zu vermeiden. Gute Katalysatoren für die $NO_x$-Reduktion sind bekanntermaßen Edelmetalle der Platinreihe, wie Platin, Palladium oder Rhodium sowie $La_{(1-x)}Sr_xCOO_3$ und $La_{(1-x)}Sr_xMnO_3$.

An den Gegenelektroden der aktiven Elektroden der Anordnung wird jeweils Sauerstoff in die umgebende Gasatmosphäre freigesetzt nach

$$2O^{2-}(ZrO_2) \rightarrow O_2 + 4e^-(El). \qquad (12)$$

Die Potentialverluste an den Gegenelektroden sollten möglichst gering sein, damit ein möglichst großer Anteil der Gesamtspannung an den aktiven Elektroden abfällt. Dies kann durch den Einsatz gemischt leitender Zwischenschichten zwischen Elektrode und Festelektrolyt erreicht werden, die bekanntermaßen die Polarisation herabsetzen.

Ein wesentlicher Vorteil der elektrokatalytischen Reaktionen ist, daß sie über die Potentialdifferenz gesteuert werden können. Dabei muß man unterscheiden zwischen Reaktionen, die ein bestimmtes Potential als treibende Kraft benötigen und Reaktionen, die in Richtung des thermodynamischen Gleichgewichts verlaufen.

In der vorgestellten Reaktionsfolge, z.B. ist NO thermodynamisch instabil gegenüber der Zersetzung zu Sauerstoff und Stickstoff, jedoch ist die Bindung im Molekül so stark, daß zur Umsetzung ein Katalysator notwendig ist. Kohlendioxid, Wasser und Schwefeldioxid dagegen sind in einem großen Partialdruckbereich stabile Komponenten. Zur Umsetzung von Kohlendioxid sind deshalb hohe kathodische Potentiale als treibende Kraft notwendig. Eine Reduktion kann dann stattfinden, wenn das Potential der Elektrode deutlich negativer als das Gleichgewichtspotential ist. Um Parallelreaktionen zur NO-Reduktion zu vermeiden, dürfen deshalb nur kathodische Potentiale bis max. -700 mV verwendet werden.

Durch die geeignete Wahl der Elektrodenmaterialien, Betriebsart der Multielektrodenanordnung, Betriebstemperatur und der einzelnen Spannungen kann eine gegenüber einzelnen Elektroden wesentlich höhere Selektivität erreicht werden. Vorgeschaltete Elektroden übernehmen dabei zu einem großen Teil die Aufgabe, Komponenten mit konkurrierenden Elektrodenreaktionen zu entfernen. Ein typisches Beispiel dafür ist die Messung von $SO_2$ bei gleichzeitiger Anwesenheit von $CO_x$ und $NO_x$. Will man $SO_2$ über die Oxidation von $SO_2$ zu $SO_3^-$ nachweisen, so müssen CO und NO aus dem Gas entfernt werden, ansonsten kommt es zu parallelen Oxidationen von CO und NO. Dies kann über eine Anordnung mit drei Elektroden erreicht werden. Die erste Elektrode dient dazu, alle Gase zu oxidieren, also CO zu $CO_2$, NO zu $NO_2$ und $SO_2$ zu $SO_3^-$ zu überführen. Dadurch wird der Einfluß des CO ausgeschaltet. An der zweiten Elektrode wird Sauerstoff, $NO_2$ und $SO_3^-$ wieder reduziert, wobei $NO_2$ bis zum Stickstoff reduziert werden muß. An der dritten Elektrode kommt von dem Gasgemisch dann nur noch, wie gewünscht, $SO_2$ an.

Parallel zur Oxidation des $SO_2$ wird an dieser Elektrode auch Sauerstoff in die Zelle gepumpt. Durch geeignete Wahl von Elektrodenmaterial, Arbeitstemperatur und Arbeitspotential kann dieser Einfluß gering gehalten werden.

Diese zwei konkreten Beispiele zeigen die prinzipiellen Möglichkeiten von derartigen Multielektrodenanordnungen für die Gasanalytik. Über ähnliche Anordnungen ist auch die Messung von CO und Kohlenwasserstoffen möglich.

Außerdem ist es möglich einen Temperatursensor zur Messung und/oder Einstellung einer geeigneten Arbeitstemperatur durch entsprechende Regelung der Heizung vorzusehen. In zweckmäßiger Ausgestaltung und Weiterbildung der Erfindung kann auch zumindest einem weiteren Sensor je ein Temperatursensor und je eine gesonderte Heizung zugeordnet werden.

Die erfindungsgemäße Anordnung, bei der der Festelektrolyt an einen Diffusionskanal angrenzt, kann auf verschiedene Weise verwirklicht werden. Beispielsweise kann eine einseitig geschlossene Meßanordnung in Form einer Rohrsonde eingesetzt werden. Das zu messende Gasgemisch gelangt über eine als Verengung ausgebildete Einströmöffnung in den Diffusionskanal. Die einzelnen Gaskomponenten werden sequentiell an den aufeinanderfolgenden Elektroden qualitativ und/oder quantitativ bestimmt.

Bei der Anordnung mit einseitig geschlossenem Raum können durch die Rückdiffusion der bei den Oxidations- und Reduktionsreaktionen entstehenden Teilchen Probleme auftreten. Deshalb erweist es sich als günstiger, in einem gasdurchströmten System mit konstantem Gasfluß zu arbeiten, das die entstehenden Teilchen abführt. Das kann z.B. über ein Pumpsystem erfolgen. Von einem derartigen System sind kürzere Ansprechzeiten zu erwarten, jedoch dürfte die vollständige Entfernung einzelner Komponenten schwieriger sein.

Aus den genannten Gründen erscheinen Anordnungen besonders vorteilhaft, bei denen das Volumen des Diffusionskanals möglichst kleingehalten wird. Dies kann mit kostengünstigen Dickschichtanordnungen mit geringem inneren Volumen realisiert werden. Als Diffusionskanal wirkt dabei eine Schicht aus poröser offenporige Keramik, die durch eine Glasschicht abgedeckt wird. Dabei kann die den Diffusionskanal bildende Schicht oberhalb der aktiven Elektroden, unterhalb der aktiven Elektroden in Form einer porösen Ionenleiterschicht oder, wie die letztere, mit einer zusätzlichen elektrischen Isolationsschicht ausgebildet sein. Dabei können auch Pulverelektroden verwendet werden. Ferner besteht auch die Möglichkeit, die Elektroden in Sandwich-Anordnung anzuordnen, wobei das obere Elektrodenpaar als Sauerstoffpumpe verwendet wird.

Die Erfindung wird nachfolgend unter Bezugnahme auf die Zeichnungen und die Beispiele näher erläutert. In diesen zeigt:

Figur 1     einige Standard-Elektrodenpotentiale und die Gleichgewichtssauerstoff-Partialdrücke einiger Reaktionen bei der Temperatur T = 900 K;

Figur 2     eine erste Ausführungsform einer erfindungsgemäßen Vorrichtung;

Figur 3     eine zweite Ausführungsform einer erfindungsgemäßen Vorrichtung;

Figur 4     eine dritte Ausführungsform der erfindungsgemäßen Vorrichtung;

Figur 5     eine vierte Ausführungsform der erfindungsgemäßen Vorrichtung;

Figur 6     eine fünfte Ausführungsform der Erfindung;

Figur 7     eine Ausführungsform mit Pulverelektroden, im schematischen Querschnitt;

Figur 8     Elektroden, in Sandwich-Anordnung und im schematischen Querschnitt;

Figur 9     der Strom einer ersten und einer zweiten Elektrode aufgetragen über der Zeit;

Figur 10    weitere Meßkurven eines Stroms einer ersten und einer zweiten Elektrode aufgetragen über der Zeit;

Figur 11    eine sechste Ausführungsform der erfindungsgemäßen Vorrichtung; und

Figur 12    eine siebte Ausführungsform .

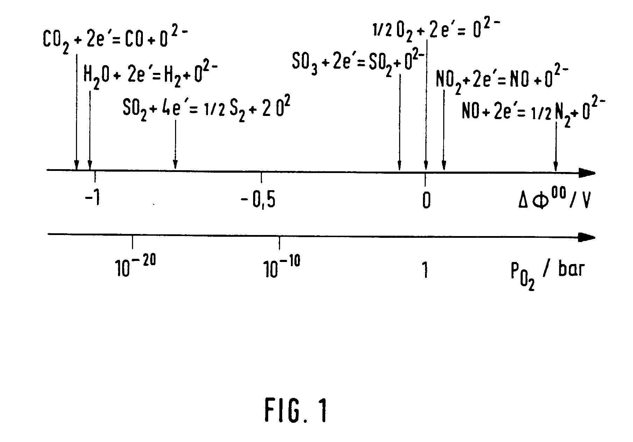

Figur 1 zeigt die Standard-Elektrodenpotentiale und die Gleichgewichtspartialdrücke einiger Reaktionen für T = 900 K und unter Annahme gleicher Konzentrationen der Gaskomponenten in der Gasphase (z.B. Verhältnis $CO/CO_2 = 1$ für das $CO/CO_2$ -Gleichgewicht). Da Kohlendioxid, Wasser und Schwefeldioxid in einem großen Partialdruckbereich stabile Komponenten sind, NO jedoch thermodynamisch instabil gegenüber der Zersetzung zu Sauerstoff und Stickstoff ist, dürfen deshalb nur kathodische Potentiale bis max. -700 mV verwendet werden, um Parallelreaktionen zur NO-Reduktion zu vermeiden.

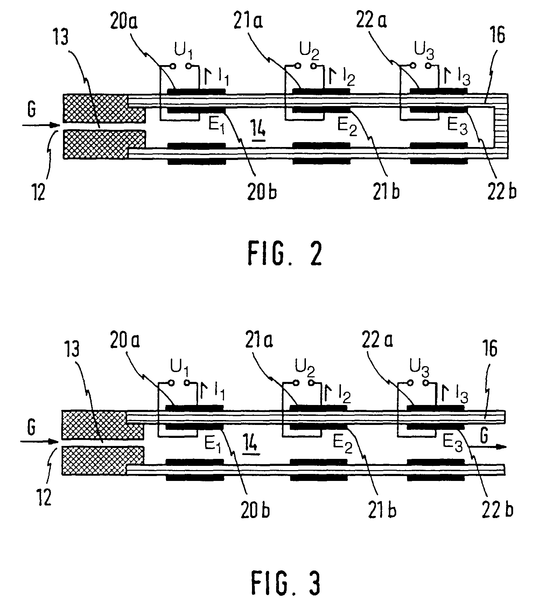

Eine Meßanordnung in Form einer Rohrsonde ist in Fig. 2 gezeigt. Die Rohrsonde besteht aus einem einseitig geschlossenem Rohr, dessen Wandung aus einem sauerstoffionenleitenden, gasdichten Festelektrolyt 16 besteht, z.B. aus stabilisiertem Zirkondioxid. Um die Wandung herum, die einen Diffusionskanal 14 bildet, sind drei Sensoren E1, E2, E3 angeordnet, deren jeweilige eine Elektrode 20b, 21b, 22b an der inneren Seite der Wandung angeordnet sind, und die jeweilige andere Elektrode 20a, 21a, 22a an der äußeren Seite der Wandung aus dem Festelektrolyten 16. Die Elektroden sind ringförmig um das Rohr herum innen und außen angeordnet. Das zu messende Gasgemisch gelangt über eine Einströmöffnung 12, die als Verengung 13 ausgebildet ist und dabei als Diffusionsbarriere wirkt, in den eigentlichen zwecks Gasdiffusion als Hohlkanal ausgebildeten Diffusionskanal 14. Die einzelnen gasförmigen Bestandteile werden sequentiell an den aufeinanderfolgenden Elektrodenpaaren E1, E2, E3 quantitativ umgesetzt.

Zur Vermeidung von durch die Rückdiffusion der bei den Oxidations- und Reduktionsreaktionen entstehenden Teilchen auftretenden Problemen kann ein Pumpsystem verwendet werden, wie es in Fig. 3 dargestellt ist. Das Pumpsystem der Fig. 3 besteht aus einem beidseitig offenen Rohr, dessen Wandung aus Festelektrolyt 16 mit der als Verengung 13 ausgebildeten Einströmöffnung 12 wie in Fig. 2 ausgestaltet sind. Auch die inneren und anderen Elektroden 20a, 20b; 21a, 21b; und 22a, 22b sind ebenso wie in Fig. 2 angeordnet.

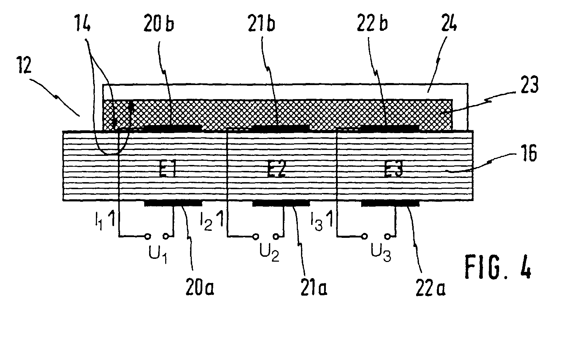

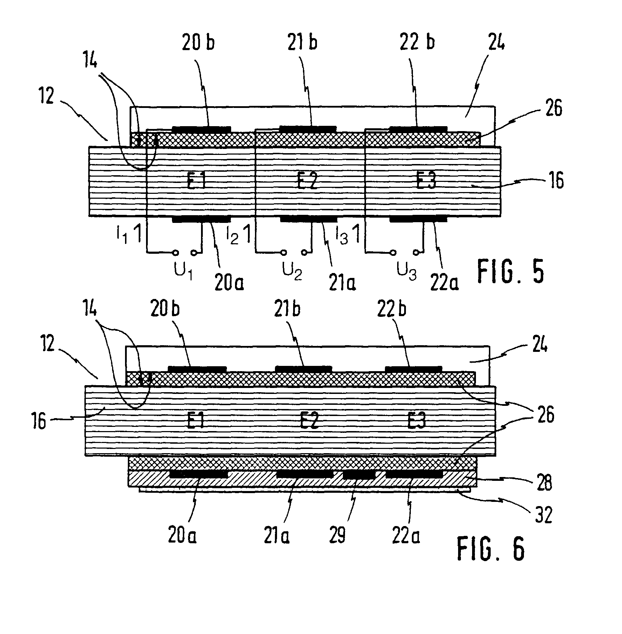

Aus den vorstehend diskutierten Gründen erscheinen Anordnungen besonders vorteilhaft, bei denen das Volumen des Diffusionskanals 14 möglichst klein gehalten wird. Dies kann mit kostengünstigen Dickschichtanordnungen mit geringem inneren Volumen realisiert werden, wie sie beispielsweise in den Fig. 4, 5 und 6 dargestellt sind. Im Diffusionskanal 14 dient dabei zum Zwecke der Gasdiffusion eine Schicht aus einem porösen offenporigen Werkstoff 23, 26, z.B. Keramik oder Glas, die mittels einer gasdichten Schicht 24, z.B. mittels einer Glasschicht abgedeckt wird. Dabei grenzt der Diffusionskanal 14 an den Festelektrolyten 16 aus sauerstoffionenleitendem, gasdichten Material und die Glasschicht 24 an ( Fig. 4 - 6). Die -aktiven- inneren Elektroden 20b, 21b, 22b können hierbei zwischen dem Diffusionskanal 14 und entweder dem Festelektrolyten 16 (Fig. 4) oder der Glasschicht 24 (Fig. 5 und 6) angeordnet sein. Bei den beiden letztgenannten Ausführungsformen müssen die an den Elektroden 20b, 21b, 22b dissoziierten Sauerstoffmoleküle als Ionen auch ein Stück durch den Festkörper des Werkstoffs 26 in dem Diffusioskanal 14 wandern, der deshalb auch ein Festelektrolyt für Sauerstoffionen sein muß, bis sie zu dem Festelektrolyten 16 gelangen. Bei allen Ausführungsformen sind die anderen Elektroden auf der dem Diffusioskanal 14 abgewandten Seite des Festelektolyten 16 angeordnet.

Die fünfte Ausführungsform gemäß Figur 6 weist auf der dem Diffusioskanal 14 abgewandten Seite des Festelektolyten 16 eine weitere Schicht aus einem porösen, offenporigen Werkstoff 26 auf, die ebenfalls an den Festelektrolyten 16 angrenzt und auf der die anderen Elektroden 20a, 21a, 22a angeordnet sind und an die sich eine zusätzliche elektrische Isolationsschicht 28 anschließt, auf der eine Heizung 32 vorgesehen ist. Zusätzlich ist ein Temperatursensor 29 zwischen der Isolationsschicht 28 und der Schicht aus dem porösen, offenporigen Werkstoff 26 vorgesehen.

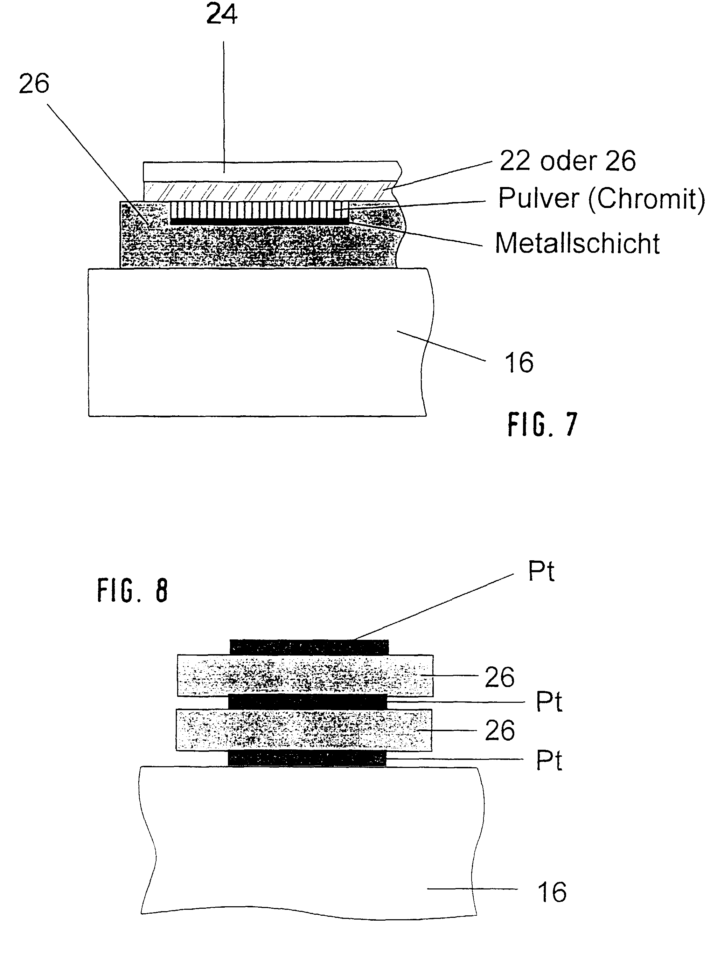

Es können auch Pulverelektroden verwendet werden. Eine entsprechende Einzelelektrode ist in Fig. 7 dargestellt. Ferner besteht auch die Möglichkeit, die Elektroden in Sandwich-Anordnung anzuordnen (Fig. 8), wobei das obere Elektrodenpaar als Sauerstoffpumpe verwendet wird.

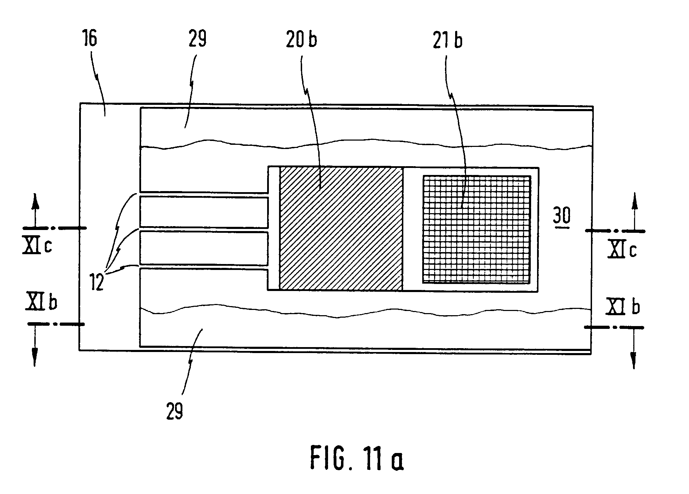

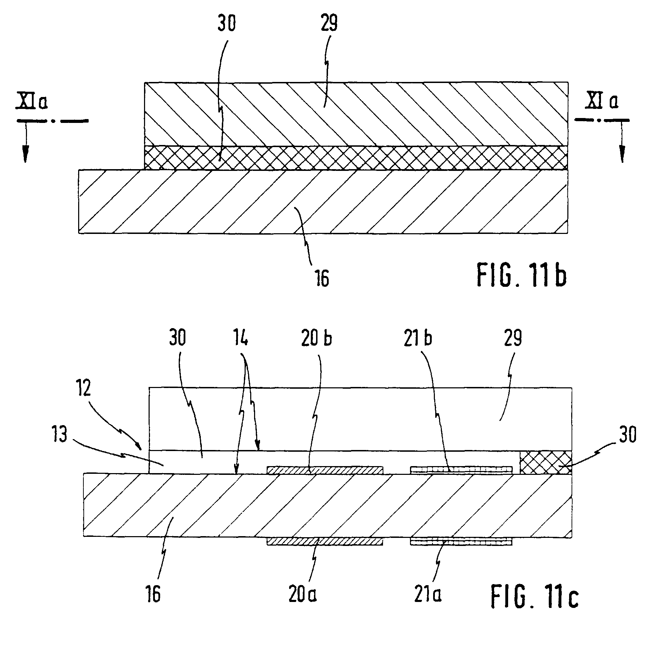

Eine weitere Ausführungsform der erfindungsgemäßen Vorrichtung ist in den Figuren 11a, b, c dargestellt. Sie enthält drei als Verengungen 13 ausgebildete Einströmöffnungen 12, die in einen als Hohlkanal zum Zweck der Gasdiffusion ausgebildeten Diffusionskanal 14 münden. Der Hohlkanal ist von einem gasundurchlässigem Werkstoff 29 in Form einer Schicht gebildet, die in dem dem Festelektrolyten abgewandten Bereich von einer weiteren Schicht aus einem ebenfalls gasundurchlässigen Werkstoff gebildet ist. Im übrigen ist diese Ausführungsform wie jene gemäß Figur 4 jedoch mit nur zwei Elektroden ausgebildet und trägt auch entsprechende Bezugszeichen.

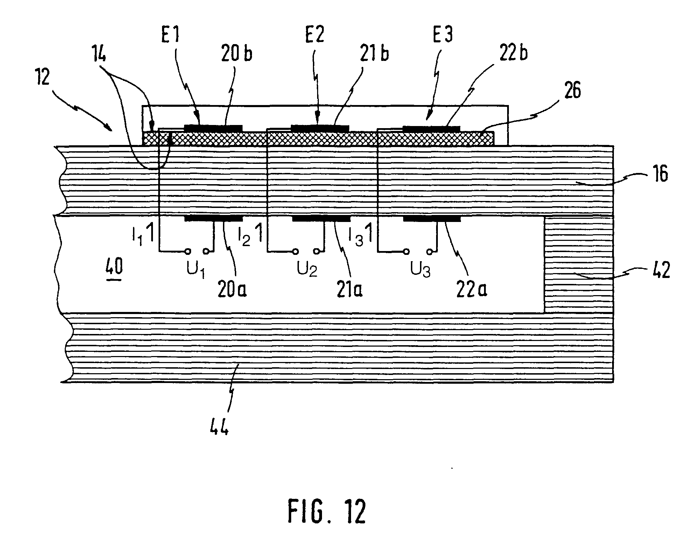

Bei der Ausführungsformg gemäß Fig. 12 ist die Anordung der Fig. 5 weitergebildet, indem unterhalb der Meßanordnung ein Gasreferenzraum 40 , z.B. für Luft angeordnet ist, der von Seitenwänden 44 und einer Endwand 42 begrenzt wird. Diese Anordnung ist insbesondere für Messungen in fetten Abgasen geeignet, die einen hohen Anteil an Kohlenwasserstoffen und/oder Kohlenmonoxid aufweisen.

Die Erfindung wird nachfolgend anhand der Beispiele näher erläutert:

BEISPIEL 1

Messung von $NO_x$ in einem sauerstoffhaltigen Gemisch

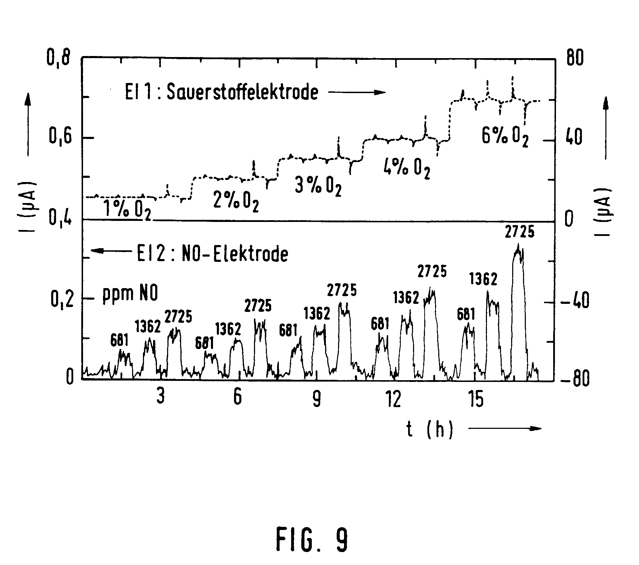

Mit einer Meßanordnung, bestehend aus einer Rohrsonde gemäß Fig. 2, jedoch mit zwei Elektrodenpaaren 20a, 20b, 21a, 21b, wobei das erste Elektrodenpaar aus Platin bestand und die zweite Elektrode 21b aus $La_{0,8}Sr_{0,2}MnO_3$ und die zweite Elektrode 21a aus Platin bestand, wurde in einem sauerstoffhaltigen Gasgemisch mit NO-Gehalt sowohl der Sauerstoffgehalt als auch der NO-Gehalt gemessen, wobei der Sauerstoffgehalt zwischen den Werten 1, 2, 3, 4 und 6 % variiert wurde. Die erhaltenen Meßkurven sind in Fig. 9 zusammengefaßt. Die obere Meßkurve EL 1 gemäß Fig. 9 gibt die gemessene Sauerstoffkonzentration wieder. Der Kurvenverlauf entspricht dem vorgegeben Anstieg der Sauerstoffkonzentration. Die untere Meßkurve EL 2 gibt die gemessene NO-Konzentration wieder und sie zeigt, daß der NO-Gehalt bei Anwesenheit von Sauerstoff meßbar ist.

BEISPIEL 2

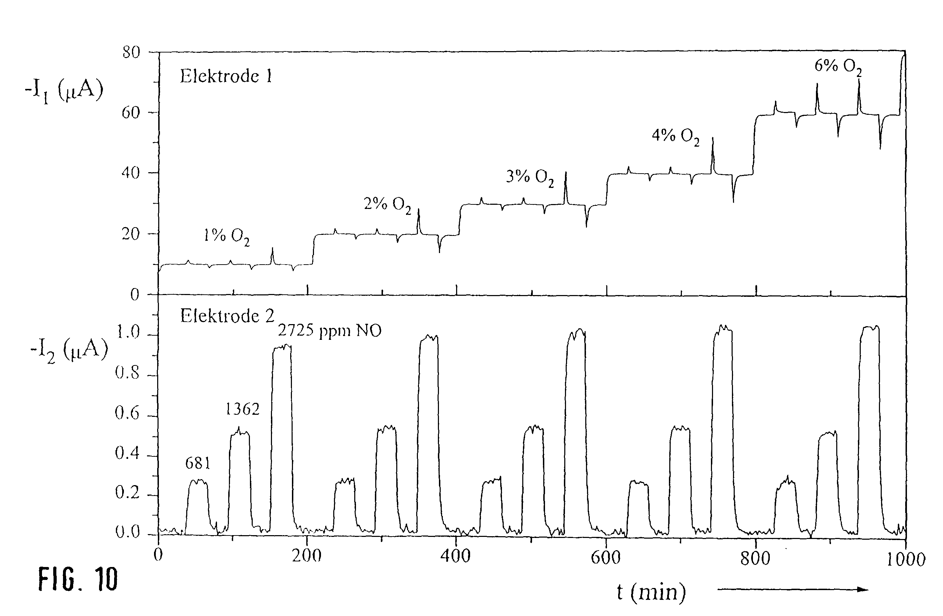

Mit einer weiteren Meßanordnung, wie jene im Beispiel 1 ist die Geometrie der Elektroden optimiert und die Sauerstoff- und NO-Konzentration unter den gleichen Bedingungen gemessen worden. Die erhaltenen Meßkurven sind in Figur 10 dargestellt. Die Optimierungen ermöglichen eine vom Sauerstoffgehalt unabhängige Bestimmung des NO-Gehaltes.

**Patentansprüche**

1. Vorrichtung zur kontinuierlichen Bestimmung der Konzentration zumindest eines gasförmigen Bestandteils von sauerstoffaufweisenden Gasgemischen mit mindestens zwei auf verschiedene gasförmige Bestandteile des Gasgemisches ansprechenden Sensoren (E1, E2), von denen jeder elektrochemische Halbzellen mit einem als Festelektrolyt (16) ausgebildeten, sauerstoffionenleitenden Material sowie vorzugsweise metall- und/oder metalloxid-haltige Elekroden (20a, 21a, 22a, 20b, 21b, 22b) als dünne Schichten aufweist, wobei durch die zwei Elektroden jedes Sensors ein von der Konzentration des von diesem gemessenen gasförmigen Bestandteil abhängiges elektrisches Meßsignal erzeugt wird und wobei der Träger ferner ggfls. mit einer elektrischen Heizung versehen ist, wobei für das Gasgemisch ein von einer Einströmöffnung (12) sich in einer Längenrichtung erstrekkender, eine Diffusionsbarriere aufweisender Diffusionskanal (14) vorgesehen ist, mit einem auf den gasförmigen Bestandteil Sauerstoff ansprechenden, amperometrisch wirkenden ersten Sensor (E1) und zumindest einem auf einen anderen gasförmigen Bestandteil als Sauerstoff ansprechenden zweiten Sensor (E2) und wobei die eine der beiden Elektroden jedes Sensors als innere Elektrode (20b, 21b) nur in dem Diffusionskanal (14) und die andere Elektrode (20a, 21a) außerhalb desselben angeordnet ist, **dadurch gekennzeichnet,** daß die mindestens zwei Sensoren (E1; E2) auf einem gemeinsamen Träger angeordnet sind, daß die Sensoren (EI, E2) in Längenrichtung des Diffusionskanals (14) nach der Einströmöffnung (12) angeordnet sind und daß die inneren Elektroden (20b, 21b) jedes Sensors (E1, E2) in direktem Kontakt mit dem im Diffusionskanal (14) befindlichen Gas gemisch stehen.

2. Vorrichtung nach Anspruch 1, dadurch gekennzeichnet, daß der Diffusionskanal (14) in Form eines Hohlkanals zur Gasdiffusion ausgebildet ist (Figuren 2, 3 und 11), wobei dieser zumindest teilweise von dem Festelektrolyten (16) begrenzt wird.

3. Vorrichtung nach Anspruch 1 oder 2, dadurch gekennzeichnet, daß der Festelektrolyt (16) gasdicht ausgebildet ist.

4. Vorrichtung nach einem der Ansprüche 1 bis 3, dadurch gekennzeichnet, daß in dem Diffusionskanal (14) ein poröser, offenporiger Werkstoff (23, 26) angeordnet ist, der zumindest teilweise quer zu dessen Längsrichtung durch gasdichtes Material (24) begrenzt ist.

5. Vorrichtung nach Anspruch 4, dadurch gekennzeichnet, daß der poröse, offenporige Werkstoff (26) in dem Diffusionskanal (14, Figuren 5, 6 und 12) zugleich als Festelektrolyt ausgebildet ist.

6. Vorrichtung nach einem der Ansprüche 1 bis 5, dadurch gekennzeichnet, daß der Diffusionskanal (14) an der der Eintrittsöffnung (12) entgegengesetzten Seite geschlossen (Figuren 2, 11 und 12) ausgebildet ist.

7. Vorrichtung nach einem der Ansprüche 1 bis 5, dadurch gekennzeichnet, daß der Diffusionskanal (14) an der der Eintrittsöffnung (12) gegenüberliegenden Seite offen (Figur 3) ausgebildet ist.

8. Vorrichtung nach einem der Ansprüche 1 bis 7, dadurch gekennzeichnet, daß die weiteren Sensoren (E2, E3...) amperometrisch oder potentiometrisch schaltbar ausgebildet ist.

9. Vorrichtung nach einem der Ansprüche 1 bis 8, dadurch gekennzeichnet, daß der Diffusionskanal (14) gänzlich von dem Festelektrolyten (16 Figuren 2 und 3) begrenzt ist.

10. Vorrichtung nach einem der Ansprüche 1 bis 9, dadurch gekennzeichnet, daß die innere Elektrode (20b) des ersten (E1) und jedes weiteren Sensors (E2, E3 ...) an dem Festelektrolyt (16) anliegt (Figuren 2 - 4 und 11).

11. Vorrichtung nach einem der Ansprüche 1 bis 10, dadurch gekennzeichnet, daß zwischen dem gasdichten Material (24) und dem Diffusionskanal (14) die inneren Elekroden (20b, 21b, 22b) angeordnet sind (Figuren 5, 6 und 12).

12. Vorrichtung nach einem der Ansprüche 1 bis 11, dadurch gekennzeichnet, daß die Eintrittsöffnung (12) des Diffusionskanals (14) als Verengung (13) ausgebildet ist (Figuren 2, 3 und 11).

13. Vorrichtung nach einem der Ansprüche 1 bis 12, dadurch gekennzeichnet, daß ein Temperaturfühler, vorzugsweise am Rand der Elektrode (20, 21, 22) zumindest einem Sensor zugeordnet ist.

14. Vorrichtung nach einem der Ansprüche 1 bis 13, dadurch gekennzeichnet, daß zumindest zwei Sensoren (E1, E2) je ein Temperaturfühler und je eine gesondert steuerbare elektrische Heizung (32) zugeordnet ist.

15. Vorrichtung nach einem der Ansprüche 1 bis 14, dadurch gekennzeichnet, daß auf der einen Seite des Festelektrolyten (16) sowohl der Temperaturfühler (29) als auch die elektrische Heizung (32) in Schichtform aufgebracht ist.

16. Vorrichtung nach einem der Ansprüche 1 bis 15, dadurch gekennzeichnet, daß der Festelektrolyt (16) stabilisiertes Zirkondioxid ($ZrO_2$) aufweist.

17. Vorrichtung nach einem der Ansprüche 1 bis 16, dadurch gekennzeichnet, daß die elektrische Heizung als Widerstandsheizschicht (32) in Form einer mäanderförmigen Bahn ausgebildet ist.

18. Vorrichtung nach einem der Ansprüche 1 bis 17, dadurch gekennzeichnet, daß die Schichten mittels Siebdruck aufgebracht sind.

19. Vorrichtung nach Anspruch 1, dadurch gekennzeichnet, daß alle Sensoren (E1, E2,...) in Längenrichtung des Diffusionskanals (14) hintereinander angeordnet sind.

20. Verfahren zur Bestimmung der Konzentration von zumindest einem gasförmigen Bestandteil in sauerstoffhaltigen Gasgemischen mit einer Vorrichtung nach einem der Ansprüche 1 bis 19, dadurch gekennzeichnet, daß mit elektrochemischen Sensoren in einem Diffusionsstrom des Gasgemisches zunächst amperometrisch der Sauerstoff bestimmt oder zumindest teilweise entfernt wird und daß danach im gleichen Diffusionsstrom des Gasgemisches zumindest eine der übrigen Komponenten amperometrisch oder potentiometrisch bestimmt wird.

21. Verfahren nach Anspruch 20, dadurch gekennzeichnet, daß die einzelnen Gaskomponenten an in Längenrichtung des Diffusionskanals (14) aufeinanderfolgenden Elektroden (20, 21, 22) sequentiell umgesetzt werden.

22. Verfahren nach Anspruch 20 oder 21, dadurch gekennzeichnet, daß Stickstoffoxide, Schwefeloxide, Kohlenmonoxid oder Kohlenwasserstoffe gemessen werden.

## Claims

1. A device for the continuous determination of the concentration at least of one gaseous component of oxygen-containing gas mixtures, with at least two sensors (EI, E2) responsive to different gaseous components of the gas mixture, each of which sensors has electrochemical half cells with an oxygen-ion conducting material formed as solid electrolyte (16), and preferably has electrodes (20a, 21a, 22a, 20b, 21b, 22b) containing metal and/or metal oxide, as thin layers, in which through the two electrodes of each sensor an electric measurement signal is generated which is dependent on the concentration of the gaseous component measured thereby, and in which the carrier in addition is provided if necessary with an electric heating, in which for the gas mixture a diffusion channel (14) is provided, extending from an inflow opening (12) in a longitudinal direction, having a diffusion barrier, with an amperometric first sensor (EI), responsive to the gaseous component oxygen, and with at least a second sensor (E2) responsive to a gaseous component other than oxygen, and in which one of the two electrodes of each sensor is arranged as inner electrode (20b, 21b) only in the diffusion channel (14) and the other electrode (20a, 21a) is arranged outside thereof, characterised in that the at least two sensors (EI; E2) are arranged on a common carrier, that the sensors (EI, E2) are arranged in longitudinal direction of the diffusion channel (14) after the inflow opening (12) and that the inner electrodes (20b, 21b) of each sensor (EI, E2) are in direct contact with the gas mixture which is in the diffusion channel (14).

2. The device according to Claim 1, characterised in that the diffusion channel (14) is constructed in the form of a hollow channel for gas diffusion (Figures 2. 3 and 11), in which the latter is at least partially delimited by the solid electrolyte (16).

3. The device according to Claim 1 or 2, characterised in that the solid electrolyte (16) is constructed so as to be gas-tight.

4. The device according to one of Claims 1 to 3, characterised in that in the diffusion channel (14) a porous, open-pored material (23, 26) is arranged, which is delimited at least partially transversely to its longitudinal direction by gas-tight material (24).

5. The device according to Claim 4, characterised in that the porous, open-pored material (26) in the diffusion channel (14, Figures 5, 6 and 12) is constructed at the same time as solid electrolyte.

6. The device according to one of Claims 1 to 5, characterised in that the diffusion channel (14) is constructed so as to be closed (Figures 2, 11 and 12) on the side opposed to the inlet opening (12).

7. The device according to one of Claims 1 to 5, characterised in that the diffusion channel (14) is constructed so as to be open (Figure 3) on the side opposite the inlet opening (12).

8. The device according to one of Claims 1 to 7, characterised in that the further sensors (E2, E3...) are constructed to be amperometrically or potentiometrically switchable.

9. The device according to one of Claims 1 to 8, characterised in that the diffusion channel (14) is entirely delimited by the solid electrolyte (16 Figures 2 and 3).

10. The device according to one of Claims 1 to 9, characterised in that the inner electrode (20b) of the first (El) and of each further sensor (E2, E3 ... ) lies against the solid electrolyte (16) (Figures 2-4 and 11).

11. The device according to one of Claims 1 to 10, characterised in that the inner electrodes (20b, 21b, 22b) are arranged between the gas-tight material (24) and the diffusion channel (14). (Figures 5, 6 and 12).

12. The device according to one of Claims 1 to 11, characterised in that the inlet opening (12) of the diffusion channel (14) is constructed as a constriction (13) (Figures 2, 3 and 11).

13. The device according to one of Claims 1 to 12, characterised in that a temperature sensor, preferably at the edge of the electrode (20, 21, 22) is associated with at least one sensor.

14. The device according to one of Claims 1 to 13, characterised in that a temperature sensor and a separately controllable electrical heating (32) are associated in each case with at least two sensors (El, E2).

15. The device according to one of Claims 1 to 14, characterised in that on the one side of the solid electrolyte (16) both the temperature sensor. (29) and also the electrical heating (32) are applied in layer form.

16. The device according to one of Claims 1 to 15, characterised in that the solid electrolyte (16) has stabilised zirconium dioxide ($ZrO_2$).

17. The device according to one of Claims 1 to 16, characterised in that the electrical heating is constructed as a resistance heating layer (32) in the form of a meandering course.

18. The device according to one of Claims 1 to 17, characterised in that the layers are applied by means of screen printing.

19. The device according to Claim 1, characterised in that all sensors (El, E2, ...) are arranged one behind the other in longitudinal direction of the diffusion channel (14).

20. A method for determination of the concentration of at least one gaseous component in oxygen-containing gas mixtures, with a device according to one of Claims 1 to 19, characterised in that firstly the oxygen is determined amperometrically with electrochemical sensors in a diffusion stream of the gas mixture or is at least partially removed, and that thereafter in the same diffusion stream. of the gas mixture at least one of the remaining components is determined amperometrically or potentiometrically.

21. The method according to Claim 20, characterised in that the individual gas components are converted sequentially on successive electrodes (20, 21, 22) in longitudinal direction of the diffusion channel (14).

22. The method according to Claim 20 or 21, characterised in that nitric oxides, sulphur oxides, carbon monoxide or hydrocarbons are measured.

**Revendications**

1. Dispositif pour la détermination continue de la concentration d'au moins un constituant gazeux de mélanges gazeux comportant de l'oxygène avec au moins deux détecteurs (E1, E2) réagissant à différents constituants gazeux du mélange gazeux, comportant chacun des demi-cellules électrochimiques avec un matériau conduisant les ions oxygène, sous forme d'électrolyte solide (16) et de préférence des électrodes contenant des métaux et/ou des oxydes métalliques (20a, 21a, 22a, 20b, 21b, 22b) sous forme de couches minces, dans lequel un signal de mesure électrique dépendant de la concentration du constituant gazeux mesuré par chaque détecteur est produit par les deux électrodes de celui-ci et dans lequel le support est éventuellement muni en outre d'un dispositif chauffant électrique, où il est prévu pour le mélange gazeux un canal de diffusion (14) comprenant une barrière de diffusion, qui s'étend depuis une ouverture d'entrée (12) dans la direction longitudinale, avec un premier détecteur (E1) à action ampérométrique qui réagit au constituant gazeux oxygène et au moins un deuxième détecteur (E2) réagissant à un autre constituant gazeux que l'oxygène et où l'une des deux électrodes de chaque détecteur est agencée sous forme d'électrode interne (20b, 21b) uniquement dans le canal de diffusion (14) et l'autre électrode (20a, 21b) est agencée à l'extérieur de celui-ci, caractérisé en ce que les deux détecteurs (E1, E2) au moins sont disposés sur un support commun, en ce que les détecteurs (E1, E2) sont disposés dans la direction longitudinale du canal de diffusion (14) après l'ouverture d'entrée (12) et en ce que les électrodes internes (20b, 21b) de chaque détecteur (E1, E2) sont en contact direct avec le mélange gazeux qui se trouve dans le canal de diffusion (14).

2. Dispositif selon la revendication 1, caractérisé en ce que le canal de diffusion (14) est agencé sous forme d'un canal creux pour la diffusion gazeuse (figures 2, 3 et 11), celui-ci étant limité au moins en partie par l'électrolyte solide (16).

3. Dispositif selon la revendication 1 ou 2, caractérisé en ce que l'électrolyte solide (16) est étanche aux gaz.

4. Dispositif selon l'une des revendications 1 à 3, caractérisé en ce qu'un matériau poreux à pores ouverts (23, 26) qui est limité par un matériau étanche aux gaz (24) au moins en partie transversalement à sa direction longitudinale est disposé dans le canal de diffusion (14).

5. Dispositif selon la revendication 4, caractérisé en ce que le matériau poreux à pores ouverts (26) dans le canal de diffusion (14, figures 5, 6 et 12) est agencé en même temps sous forme d'électrolyte solide.

6. Dispositif selon l'une des revendications 1 à 5, caractérisé en ce que le canal de diffusion (14) est fermé (figures 2, 11 et 12) du côté opposé à l'ouverture d'entrée (12).

7. Dispositif selon l'une des revendications 1 à 5, caractérisé en ce que le canal de diffusion (14) est ouvert du côté opposé à l'ouverture d'entrée (12) (figure 3).

8. Dispositif selon l'une des revendications 1 à 7, caractérisé en ce que les autres détecteurs (E2, E3, ...) sont agencés de manière à pouvoir être montés ampérométriquement ou potentiométriquement.

9. Dispositif selon l'une des revendications 1 à 8, caractérisé en ce que le canal de diffusion (14) est totalement limité par l'électrolyte solide (16) (figures 2 et 3).

10. Dispositif selon l'une des revendications 1 à 9, caractérisé en ce que l'électrode interne (20b) du premier détecteur (EI) et de chaque autre détecteur (E2, E3, ...) est contiguë à l'électrolyte solide (16) (figures 2-4 et 11).

11. Dispositif selon l'une des revendications 1 à 10, caractérisé en ce que les électrodes internes (20b, 21b, 22b) sont disposées entre le matériau étanche aux gaz (24) et le canal de diffusion (14) (figures 5, 6 et 12).

12. Dispositif selon l'une des revendications 1 à 11, caractérisé en ce que l'ouverture d'entrée (12) du canal de diffusion (14) est agencée sous forme d'un rétrécissement (13) (figures 2, 3 et 11).

13. Dispositif selon l'une des revendications 1 à 12, caractérisé en ce qu'un capteur de température est associé à au moins un détecteur, de préférence au bord des électrodes (20, 21, 22).

14. Dispositif selon l'une des revendications 1 à 13, caractérisé en ce qu'un capteur de température et un dispositif chauffant électrique (32) qui peut être commandé séparément sont associés dans chaque cas à au moins deux détecteurs (EI, E2).

15. Dispositif selon l'une des revendications 1 à 14, caractérisé en ce que le capteur de température (29) et le dispositif chauffrant électrique (32) sont appliqués sous forme d'une couche sur un côté de l'électrolyte solide (16).

16. Dispositif selon l'une des revendications 1 à 15, caractérisé en ce que l'électrolyte solide (16) comporte du dioxyde de zirconium ($ZrO_2$) stabilisé.

17. Dispositif selon l'une des revendications 1 à 16, caractérisé en ce que le dispositif chauffant électrique est agencé à la manière d'une couche chauffante résistive (32) sous forme d'une bande formant des ondulations.

18. Dispositif selon l'une des revendications 1 à 17, caractérisé en ce que les couches sont appliquées par sérigraphie.

19. Dispositif selon la revendication 1, caractérisé en ce que tous les détecteurs (E1, E2,...) sont agencés les uns derrière les autres dans la direction longitudinale du canal de diffusion.

20. Procédé pour déterminer la concentration d'au moins un constituant gazeux dans des mélanges gazeux contenant de l'oxyène avec un dispositif selon l'une des revendications 1 à 19, caractérisé en ce qu'avec des détecteurs électrochimiques dans un courant de diffusion du mélange gazeux l'oxygène est tout d'abord déterminé ampérométriquement ou est éliminé au moins en partie et en ce qu'ensuite, dans le même courant de diffusion du mélange gazeux, au moins l'un des autres composants est déterminé ampérométriquement ou potentiométriquement.

21. Dispositif selon la revendication 20, caractérisé en ce que les différents composants gazeux sont convertis successivement au niveau d'électrodes (20, 21, 22) consécutives dans la direction longitudnale du canal de diffusion (14).

22. Procédé selon la revendication 20 ou 21, caractérisé en ce que des oxydes d'azote, des oxydes de soufre, du monoxyde de carbone ou des hydrocarbures sont mesurés.

$CO_2 + 2e' = CO + O^{2-}$

$H_2O + 2e' = H_2 + O^{2-}$

$SO_2 + 4e' = 1/2 S_2 + 2 O^2$

$1/2 O_2 + 2e' = O^{2-}$

$SO_3 + 2e' = SO_2 + O^{2-}$

$NO_2 + 2e' = NO + O^{2-}$

$NO + 2e' = 1/2 N_2 + O^{2-}$

$-1$  $-0,5$  $0$  $\Delta \Phi^{00} / V$

$10^{-20}$  $10^{-10}$  $1$  $P_{O_2} / bar$

FIG. 1

FIG. 2

FIG. 3

FIG. 4

FIG. 5

FIG. 6

24

26

22 oder 26

Pulver (Chromit)

Metallschicht

16

FIG. 7

FIG. 8

Pt

26

Pt

26

Pt

16

FIG. 9

FIG. 10

FIG. 11 a

XI a

30    29

XI a

16

FIG. 11b

30   14   20 b   21 b   29

12

13

30

16   20a   21a   FIG. 11c

FIG. 12